(19) Europäisches Patentamt
European Patent Office
Office européen des brevets

(11) Veröffentlichungsnummer: **0 252 875 B1**

(12) **EUROPÄISCHE PATENTSCHRIFT**

(45) Veröffentlichungstag der Patentschrift: **27.01.93**

(51) Int. Cl.[5]: **A01N 43/653**, C07C 309/28, C07D 265/30, C07D 295/02

(21) Anmeldenummer: **87810319.1**

(22) Anmeldetag: **02.06.87**

# (54) Mikrobizide Mittel.

(30) Priorität: **07.06.86 GB 8613913**

(43) Veröffentlichungstag der Anmeldung:
**13.01.88 Patentblatt 88/02**

(45) Bekanntmachung des Hinweises auf die Patenterteilung:
**27.01.93 Patentblatt 93/04**

(84) Benannte Vertragsstaaten:
**AT BE CH DE ES FR GB GR IT LI LU NL SE**

(56) Entgegenhaltungen:
**EP-A- 0 074 329**
**US-A- 2 687 397**
**US-A- 3 642 995**

(73) Patentinhaber: **CIBA-GEIGY AG**
**Klybeckstrasse 141**
**CH-4002 Basel(CH)**

(72) Erfinder: **Nowak, Edward**
**12 Alice Way, Histon**
**Cambridge CB4 4YA(GB)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Morpholinsalze der Benzolsulfonsäure und alkylierter Benzolsulfonsäuren; Verfahren zu deren Herstellung; und deren Verwendung in fungiziden Mitteln zur Bekämpfung schädlicher Mikroorganismen, insbesonderen phytopathogener Pilze.

Aus der GB-PS 1522657 sind mikrobizide Mittel bekannt geworden, welche als Aktivsubstanz eine Verbindung der Formel I

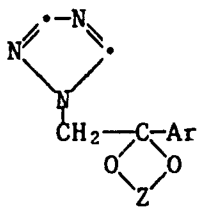

I

oder ein Säureadditionssalz dieser Verbindung enthalten;
worin
Z $-CH_2CH_2-$, $-CH_2CH_2CH_2-$, $-CH(CH_3)-CH(CH_3)-$ oder $-CH_2-CH(C_1-C_{10}\text{-alkyl})-$; und
Ar einen Thiophenyl-, Halogenthiophenyl-, Naphthalinyl- oder einen unsubstituierten oder gegebenenfalls bis zu dreifach durch Halogen, $C_1$-$C_4$-Alkyl, $C_1$-$C_4$-Alkoxy, Cyano oder Nitro substituierten Phenylrest bedeutet.

Die Verbindungen der Formel I sind hoch wirksame fungizide Wirkstoffe und können sowohl alleine wie auch in Kombination mit anderen bekannten Fungiziden wie etwa Tridemorph oder Carbendazim in vorteilhafter Weise verwendet werden.

Im der US-A-2 687 397 sind einige Morpholimsalze der Benzolsulfonsäure beschrieben.

Es ist bekannt, die Verbindung der Formel I sowohl alleine wie auch in Kombination mit anderen Fungiziden in konventioneller Weise unter Verwendung organischer Lösungsmittel als emulgierbare Konzentrate zu formulieren. Darüber hinaus ist aus der EP-A-74329 bekannt geworden. Verbindungen der Formel I unter Verwendung eines Hydrotrops, z.B. Morpholimium benzolsulfonaten, als wässrige Lösungskonzentrate zu formulieren. Hydrotrope sind wasserlösliche Verbindungen, welche die Löslichkeit einer Zweiten, in Wasser an sich nicht oder nur schwer löslichen Komponente erhöhen (Chemiker Zeitung; 95 - (1971) 507-511). Darüber hinaus ist die Verwendung hydrotroper Aktivsubstanzen bei agrochemischen Formulierungen von Mikroemulsionen bekannt (FR-A-2187227, FR-A-2187226). Hydrotrope sind u.a. die Alkalimetall- oder Ammoniumsalze niederer Alkylbenzolsulfonsäuren, wie etwa das Natriumsalz der Toluolsulfonsäure, das Natriumsalz der Xylosulfonsäure und das Ammoniumsalz der Cumolsulfonsäure. Diese an sich bekannten Hydrotrope verbessern nicht die Fungizidwirkung einer Fungizidformulierung, in welcher sie enthalten sind.

Es wurde nun überraschenderweise gefunden, dass die fungizide Wirkung von Verbindungen der Formel I in Kombination mit bestimmten hydrotropen Aktivsubstanzen eine verbesserte Mikrobizidwirkung im Vergleich zu den aus dem Stand der Technik bekannten Hydrotropformulierungen zeigt.

Die Erfindung betrifft somit mikrobizide Mittel bestehend aus einer Verbindung der Formel I und einer synergistisch wirksamen Menge eines Salzes der Formel II

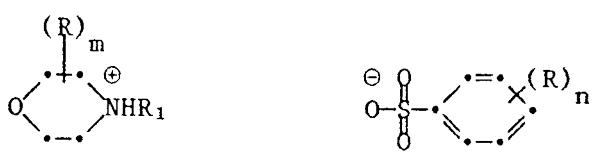

II,

in welcher
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet.

Insbesondere betrifft die Erfindung mikrobizide Zusammensetzungen enthaltend als Verbindung der Formel I:
1-[2-(2,4-Dichlorophenyl)-1,3-dioxolan-2-ylmethyl]-1H-1,2,4-triazol;
1-[2-(2,4-Dichlorophenyl)-4-methyl-1,3-dioxolan-2-ylmethyl]-1H-1,2,4-triazol;
1-[2-(2,4-Dichlorophenyl)-4-ethyl-1,3-dioxolan-2-ylmethyl]-1H-1,2,4-triazol;
1-[2-(2,4-Dichlorophenyl)-4-propyl-1,3-dioxolan-2-ylmethyl]-1H-1,2,4-triazol (Propiconazol); und
1-[2-(2,4-Dichlorophenyl)-4-pentyl-1,3-dioxolan-2-ylmethyl]-1H-1,2,4-triazol;
sowie die als agrochemischen Formulierungen verwendbaren Säureadditionssalze dieser Verbindungen; wie z.B. die Salze anorganischer Säuren, wie Halogenwasserstoffsäure, Schwefelsäure, Salpetersäure oder Phosphorsäure oder die Salze organischer Säure, wie Essigsäure, Propionsäure oder Malonsäure. Als besonders bevorzugte Verbindung der Formel I ist Propiconazol zu nennen. Verfahren zur Herstellung von Verbindungen der Formel I sind in der GB-PS 1522657 beschrieben.

Als Salze der Formel II sind insbesondere zu nennen:
Morpholin Benzolsulfonat,
Morpholin Toluolsulfonat,
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Xylolsulfonat,
2,6-Dimethylmorpholin Benzolsulfonat,
2,6-Dimethylmorpholin Xylolsulfonat,
2,2,6-Trimethylmorpholin Cumolsulfonat,
2,2,6,6-Tetramethylmorpholin Cumolsulfonat,
besonders bevorzugt sind Morpholin Cumolsulfonate wie
N-Methylmorpholin Cumolsulfonat und
2,6-Dimethylmorpholin Cumolsulfonat.

Die erfindungsgemässen Morpholinsalze der Formel II sind teilweise neu. Das Morpholinsalz der Benzolsulfonsäure und der 4-Toluolsulfonsäure sind bereits in der wissenschaftlichen Literatur beschrieben (Beilstein, Handbuch der Org. Chemie; Bd. 27 , 21). Weitere Salze sind in der US-A-2 687 397 beschrieben. Die noch nicht zu dem Stand der Technik gehörenden Verbindungen der Foreml II bilden einen weiteren Gegenstand der Erfindung.

Die Erfindung betrifft somit auch die neuen Salze der Formel II:

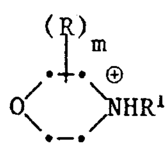 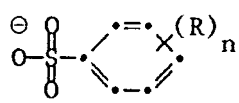 II,

worin
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet, mit der Massgabe, dass
2,6-Dimethylmorpholin Benzolsulfonat
2,6-Dimethylmorpholin o-Toluolsulfonat
2,6-Dimethylmorpholin m-Toluolsulfonat
2,6-Dimethylmorpholin p-Toluolsulfonat
2,6-Dimethylmorpholin 2,4,6-Trimethylbenzolsulfonat
N-Butylmorpholin Benzolsulfonat
N-Butylmorpholin o-Toluolsulfonat
N-Butylmorpholin m-Toluolsulfonat
N-Butylmorpholin p-Toluolsulfonat und
N-Butylmorpholin 2,4,6-Trimethylbenzolsulfonat nicht umfasst sind.

Bevorzugte Salze der Formel II sind:
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Cumolsulfonat,
2,6-Dimethylmorpholin Cumolsulfonat,
2,2,6-Trimethylmorpholin Cumolsulfonat,
2,2,6,6-Tetramethylmorpholin Cumolsulfonat,
besonders bevorzugt sind die Morpholincumolsulfonate der Formel II mit m = 0, 1, 2, 3 oder 4 und R = $CH_3$ oder $C_2H_5$ und $R^1$ = $CH_3$ oder $C_2H_5$ insbesondere das N-Methylmorpholin Cumolsulfonat und 2,6-Dimethylmorpholin Cumolsulfonat.

Die Salze der Formel II können durch Umsetzung eines gewünschtenfalls substituierten Morpholins der Formel III

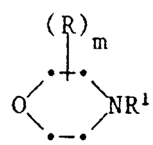

III,

in der
R unabhängig voneinander $C_1$-$C_4$-Alkyl,
$R^1$ Wasserstoff oder $C_1$-$C_4$-Alkyl und
m 0, 1, 2, 3 oder 4 bedeutet, mit einer Sulfonsäure der Formel IV

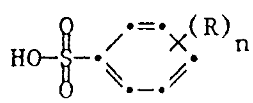

IV

in der
R unabhängig voneinander $C_1$-$C_4$-Alkyl, und
n 0, 1, 2, 3 oder 4 bedeutet,
in an sich bekannter Weise gewonnen werden. Die so erhältlichen Salze der Formel II können sowohl in fester als auch in gelöster (insbesondere in wässriger Lösung) Form erhalten werden.

Die erfindungsgemässe Reaktion wird vorzugsweise durch Umsetzung im wesentlichen stöchiometrischer Mengen der Verbindungen der Formel III und IV bei Temperaturen zwischen Raumtemperatur und 100 ° C durchgeführt. Die Reaktion kann in einem Lösungsmittel wie z.B. Wasser, Ethylacetat, Isopropylalkohol oder Ethanol durchgeführt werden. Die Wahl des jeweils geeigneten Lösungsmittels ist insbesondere davon abhängig, ob man das Salz der Formel II in Lösung oder in kristalliner Form isolieren möchte. Sofern die Isolierung als Feststoff gewünscht wird, ist es vorteilhaft, die Umsetzung der Verbindungen III und IV in einem Lösungsmittel vorzunehmen, in welchem die Verbindungen III und IV für sich löslich sind, das Salz II jedoch unlöslich oder schwer löslich ist. In besonders vorteilhafter Weise können, wie später im einzelnen erläutert wird, die Salze der Formel II als Hydrotrope zur Herstellung wässriger Lösungskonzentrate von Verbindungen der Formel I Verwendung finden. In diesem Falle ist es vorteilhaft, die Umsetzung der Edukte III und IV in Wasser durchzuführen; die als direktes Reaktionsprodukt erhältliche wässrige Lösung des Salzes II kann ohne weitere Aufarbeitung zur Herstellung der erfindungsgemässen Hydrotropformulierung Verwendung finden.

Die erfindungsgemässen Mittel haben ein verbessertes mikrobizides Wirkspektrum gegen phytopathogene Pilze und Bakterien, insbesondere gegen Erysiphe Graminis (Mehltau). Aufgrund ihrer sehr vorteilhaften kurativen, systemischen und teilweise präventiven Eigenschaften können sie zum Schutze zahlreicher Kulturpflanzen Verwendung finden. Mit den erfindungsgemässen Mitteln ist es möglich, den Befall von Mikroorganismen, welcher an Pflanzen oder Teile von Pflanzen (Frucht, Blüte, Blatt, Stamm, Stiel, Knollen, Wurzeln) auftreten kann, in einem solchen Ausmass zu bekämpfen oder zu inhibieren, dass auch die nach

dem Befall nachwachsenden Pflanzenteile vor dem Angriff des Mikroorganismus geschützt sind.

Die erfindungsgemässen Mittel sind inbesondere gegen die nachstehend angeführten Klassen phytopathogener Pilze wirksam: Fungi imperfecti (z.B. Botrytis, Helminthosporium, Fusarium, Septoria, Cereospora und Alternaria); Basidiomyceten (z.B. der Gattung Hemileia, Rhizocotonia, Puccinia); und insbesondere gegen Ascomyceten (z.B. Venturia, Podosphaera, Erysiphe, Molinia, Uncinula). Ausserdem können die erfindungsgemässen Mittel auch als Saatbeizmittel zum Schutze von Fortpflanzungsgut (Früchte, Knollen, Samenkörner) und auch zum Schutze von Pflanzenteilen gegen Pilzinfektionen und gegen phytopathogene Mikroorganismen, welche im Boden vorkommen, verwendet werden.

Ein weiterer Gegenstand der Erfindung ist ein Verfahren zur Herstellung agrochemischer Mittel, welches dadurch gekennzeichnet ist, dass man die Aktivsubstanzen der Formel I und II mit einem oder mehreren der nachstehend beschriebenen Hilfs-und/oder Streckmittel in inniger Weise vermischt. Die Erfindung betrifft ausserdem ein Verfahren zur Behandlung von Pflanzen, dadurch gekennzeichnet, dass man ein erfindungsgemässes Mittel auf die Pflanze oder deren Lebensraum einwirken lässt. Bei beiden Verfahren sind Verbindungen der Formel II ausgeschlossen, worin $R_1$ gleich Wasserstoff ist und $m = 0$ ist, wenn es sich bei dem Streckmittel um Wasser handelt. Die nachstehend genannten Kulturpflanzen können in besonders vorteilhafter Weise mit dem erfindungsgemässen Mittel behandelt werden: Getreide (Weizen, Gerste, Roggen, Hafer, Reis, Sorghum und verwandte Kulturen); Rüben (Zuckerrübe und Futterrübe); Steinfrüchte, Kernfrüchte und kernlose Früchte (Aepfel, Birnen, Pflaumen, Pfirsiche, Mandeln, Kirschen, Erdbeeren, Himbeeren, Brombeeren, etc.); Leguminosen (Bohnen, Linsen, Erbsen, Soja); Oelpflanzen (Raps, Senf, Mohn, Oliven, Sonneblumen, Kokos, Rizinuspflanzen, Kakao, Erdnüsse); Gurkengewächse (Gurke, Kürbis, Melone); Faserpflanzen (Baumwolle, Flachs, Sisal, Jute); Zitrusfrüchte (Orangen, Zitronen, Grapefruit, Mandarinen); Gemüse (Spinat, Lattich, Salat, Spargel, Kohl, Karotten, Zwiebeln, Tomaten, Kartoffeln, Paprika); Lauraceae (Avocados, Zimt, Kampher); oder Pflanzen wie Mais, Tabak, Nüsse, Kaffee, Zuckerrohr, Tee, Wein, Hopfen, Bananen sowie Gummigewächse, Blumen (Kornblütler); vorzugsweise Getreide, insbesondere Weizen (Winter-und frühe Arten) und Gerste (Winter- und frühe Arten).

Die erfindungsgemässen Mittel können gemeinsam oder nacheinander mit weiteren Agrochemikalien auf die Pflanze oder die zu behandelnden Fläche aufgebracht werden. Als Agrochemikalien sind in erster Linie zu nennen Dünger oder Spurennährstoffe oder andere Zubereitungen welche das Pflanzenwachstum beeinflussen. Des weiteren zu nennen sind selektive Herbizide; Fungizide, insbesondere Tridemorph, Fenpropimorph und/oder Carbendazim; Bakterizide; Nematizode; Molluskizide oder Mischung der vorgenannten Wirkstoffklassen mit weiteren Trägern, oberflächenaktiven Mitteln, oder sonstigen an sich bekannte, die Applikation begünstigende, Hilfsstoffe. Es können sowohl feste wie auch flüssige Träger- und Hilfsstoffe verwendet werden, sie sind in der Formulierungstechnik allgemein bekannt, wie etwa natürliche oder regenerierte Mineralstoffe. Lösungsmittel, Dispergiermittel, Netzmittel, Verdicker, Bindemittel oder Dünger.

In vorteilhafter Weise können die erfindungsgemässen Mittel auf die Kulturpflanze durch Blattapplikation aufgebracht werden. Die Anzahl der Behandlungen sowie die Applikationsrate ist von der Befallstärke und dem Infektionsdruck des jeweils zu bekämpfenden Pathogens (Pilzart) abhängig. Durch Aufbringung auf den Wuchsort der Pflanze kann die erfindungsgemässe Zubereitung von der Pflanze auch durch ihre Wurzeln aus dem Boden aufgenommen und ihre Wirkung in den oberirdischen Teilen der Pflanze entfalten (systemische Wirkung). Die erfindungsgemässen Mittel können auch durch Imprägnierung des Vermehrungsguts (insbesondere Samen und Knollen) von Kulturpflanzen als Samenbeizmittel verwendet werden. Das so erhältliche mit den erfindungsgemässen Mitteln gebeizte Vermehrungsgut ist ebenfalls Gegenstand der Erfindung. Spezielle, gezielte Applikationsformen sind auch möglich, wie etwa das selektive Behandeln von Pflanzenstielen, Stämmen oder Knospen.

Die erfindungsgemässen Mittel können in an sich bekannter Weise zu emulgierbaren Konzentraten, Pasten, direkt sprühbar oder verdünnbare Lösungen, verdünnte Emulsionen, Suspensionskonzentraten, netzbaren Pulvern oder in Wasser dispergierbaren Granulaten formuliert werden. Die günstigste Ausbringungsform, wie Sprühen, Benetzen, Verstäuben, Vernebeln, Tränkon etc., wird unter Berücksichtigung der in der Zusammensetzung enthaltenden Bestandteile sowie unter Berücksichtigung des jeweils angestrebten Behandlungszwecks frei gewählt.

Im allgemeinen liegen die Aufwandmengen zwischen 50 g und 5 kg Aktivsubstanz/ha, vorzugsweise von 100 g bis 2 kg Aktivsubstanz/ha, insbesondere von 200 bis 600 g Aktivsubstanz/ha.

Durch homogenes Mischen und/oder Malen der Aktivsubtanzen mit Streckmitteln, wie z.B. Lösungsmittel und gegebenenfalls oberflächenaktiven Mitteln, werden die erfindungsgemässen Mittel in an sich bekannter Weise hergestellt.

Geeignete Lösungsmittel sind: aromatische Kohlenwasserstoffe, vorzugsweise Fraktionen mit 8 bis 12 Kohlenstoffatomen, wie z.B. Xylole oder substituierte Naphthaline, Phthalate, wie etwa Dibutylphthalat oder Dioctylphthalat; aliphatische Kohlenwasserstoffe, wie Cyclohexan oder Paraffine; Alkohole und Glycole und

deren Ether und Ester, wie etwa Ethanol, Ethylenglycolmonomethyl- und -monoethylether; Ketone, wie Cyclohexanon; polare Lösungsmittel wie N-Methyl-2-pyrrolidon, Dimethylsulfoxid oder Dimethylformamid; epoxidierte Pflanzenöle, wie epoxidiertes Kokonuss- oder Sonnenblumenöl; oder Wasser.

Die in der Formulierungstechnik gebräuchlichen oberflächenaktiven Mittel und Hilfsstoffe sind beispielsweise beschrieben in "1985 International Mc. Cutcheon's Emulsifiers and Detergents, Glen Rock, NY 07452 USA" sowie in der "Encyclpedia of Surface Active Agents, Chemical Publishing Co., Inc. New York 1980". Oberflächenaktive Verbindungen sind nichtionische, amphothere und/oder anionaktive Mittel mit guter Emulgier-, Dispergier- und Netzwirkung. Der Begriff "oberflächenaktive Mittel" umfasst auch Mischungen einzelner oberflächenaktiver Mittel.

Geeignete anionenaktive Mittel können sowohl wasserlösliche Seifen wie auch wasserlösliche synthesische oberflächenaktive Mittel sein.

Vorzugsweise werden in den erfindungsgemässen Mitteln nicht ionische oberflächenaktive Stoffe verwendet. Als nicht ionische Tenside kommen in erster Linie Polyglykolether-und -esterderivate von aliphatischen oder cycloaliphatischen Alkoholen, von gesättigten oder ungesättigten Fettsäuren und von Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglycol mit 1 bis 10 Kohlenstoffatomen in der Alkylkette, die genannten Verbindungen enthalten üblicherweise pro Propylenglykol-Einheit 1 bis 5 Ethylenglykoleinheiten.

Die erfindungsgemässen mikrobiziden Mittel enthalten neben einem Triazolderivat der Formel I eine für eine synergistische Wirkungssteigerung ausreichende Menge eines Salzes der Formel II. Insbesondere enthalten die mikrobiziden Mittel 0,1 bis 99 %, vorzugsweise 0,1 bis 95 %, einer Verbindung der Formel I und 99,9 bis 1 %, vorzugsweise 99,8 bis 5 % eines Salzes der Formel II sowie gewünschtenfalls 0 bis 25 % eines oberflächenaktiven Mittels und 0 bis 25 % eines Lösungsmittels.

Während die Handelsprodukte üblicherweise in konzentrierter Form verwendet werden, wird der Endverbraucher die in üblicher Weise auf Anwendungskonzentration verdünnten Formulierungen verwenden. In besonders vorteilhafter Weise können analog zu den aus der EP-A-74329 bekannten Hydrotropformulierungen, auf die hier ausdrücklich Bezug genommen wird, wässrige Lösungskonzentrate der erfindungsgemässen Mittel hergestellt werden. Dabei dienen die Salze der Formel II als hydrotrope Aktivsubstanzen, während die Triazolderivate der Formel I als die im wesentlichen in Wasser unlöslichen Komponente in einer wässrigen Lösung des Salzes der Formel II gelöst werden. Die hydrotropen Eigenschaften der Salze der Formel II können in besonders vorteilhafter Weise zur Herstellung wässriger Lösungskonzentrate mit bis zu 75 Gew.-%/Vol.-% an Aktivsubstanz der Formel I in bis zu 70 Gew.-%/Vol.-%-igen wässrigen Lösungen des Hydrotrops der Formel II enthalten. Bevorzugt sind Lösungskonzentrate, welche durch die Lösung von 5 bis 50 Gew.-%/Vol.-% einer Verbindung der Formel I in einer 5 bis 70 Gew.-%/Vol.-%-igen wässrigen Lösung von II erhältlich sind.

Die nachstehenden Beispiele erläutern den Erfindungsgegenstand.

Beispiel 1: 20 Teile Cumolsulfonsäure (Eltesol CA 96, ex Albright and Wilson)werden in der gleichen Menge Ethanol gelöst. Zu dieser Lösung werden bei 20 °C 8,7 Teile Morpholin gegeben, die Temperatur wird dabei unterhalb von 35 °C gehalten. Nach Beendigung der Morpholinzugabe wird noch 15 Minuten weitergerührt, und dann auf -5 °C gekühlt, wobei Morpholin Cumolsulfonat auskristallisiert. Nach 24 Stunden wird abfiltriert, dreimal mit kaltem Diethylether gewaschen und in einem Exsiccator über Silicagel getrocknet. Der Schmelzpunkt des so erhältlichen Morpholin Cumolsulfonats beträgt 101-105 °C.

Beispiel 2: 20 Gewichtsteile Cumolsulfonsäure werden in der gleichen Menge Wasser gelöst. Unter Rühren und Kühlen auf 20 °C werden 11,6 Gewichtsteile 2,6-Dimethylmorpholin (99 % rein) zugetropft, wobei die Temperatur unterhalb von 35 °C gehalten wird. Danach wird die Lösung durch Zugabe weiteren deionisierten Wassers bis zu einer 60 Gew.-%igen Lösung von 2,6-Dimethylmorpholin Cumolsulfonat (DMMCS) verdünnt.

Beispiel 3: Propiconazol 125 EC (emulgierbares Konzentrat)

| | [g/l] |
|---|---|
| Propiconazol | 125 |
| 2,6-Dimethylmorpholin Cumolsulfonat | 580 |
| Wasser | ca. 390 |

Das 2,6-Dimethylmorpholin Cumolsulfonat (DMMCS) wird in der angegebenen Menge Wasser gelöst. Danach wird die angegebene Menge Propiconazol zugesetzt und das gesamte Gemisch gerührt bis eine homogene Lösung erreicht ist.

Beispiel 4: Propiconazol 250 EC

| | [g/l] |
|---|---|
| Propiconazol | 250 |
| Cyclohexan | 100 |
| $C_{10}$-Alkoholethoxylat (Ethylan CD 913), Warenzeichen der Lankro Chemicals Ltd, Manchester, GB) | 30 |
| Ethylenoxid/Propylenoxid Blockcopolymer (Pluronic F 108, Warenzeichen der BASF Wyandotte Corp. USA) | 15 |
| Morpholin Cumolsulfonat | 420 |
| Wasser | ca. 270 |

Das Morpholin Cumolsulfonat wird in Wasser gelöst, anschliessend wird das Alkoholethoxylat und das Ethylenoxid/Propylenoxid Blockcopolymer zugegeben. Danach wird mit dem Cyclohexanon und dem Propiconazol versetzt und die gesamte Mischung geschüttelt bis eine homogene Lösung erreicht ist.

Beispiel 5: Propiconazol + Carbendazim 225 SC (Suspensionskonzentrat)

| | [g/l] |
|---|---|
| Propiconazol | 125 |
| Carbendazim | 100 |
| Ethoxyliertes Polyarylphenolphosphat (Soprophor FL, ex Rhone Poulenc) | 60 |
| Antischaummittel auf Siliconbasis (Foammaster UDB) | 5 |
| Kieselgur (Aerosil COK-84 ex Degussa) | 10 |
| 2,6-Dimethylmorpholin-Cumolsulfonat (DMMCS) | 470 |
| Wasser | ca. 330 |

Das 2,6-Dimethylmorpholin Cumolsulfonat wird in Wasser gelöst und danach mit dem ethoxyliereten Polyarylphenolphosphat und Propiconazol versetzt. Unter Rühren mit einem Magnetrührer wird sodann das Antischaummittel, das Carbendazim und das Kieselgur darin dispergiert. Die so erhältliche Suspension wird bis zu einer Teilchengrösse von 2 Mikron nass vermahlen.

Beispiele 5 und 6: Die Verbesserung der Mehltauwirkung mittels der erfindungsgemässen Mittel wird in einer 5-reihigen Testserie in einem Gewächshaus-Versuch demonstriert.

In Schalen angezogene Schösslinge der Frühgersten-Sorte "Golden Promise" werden in einem Gewächshaus mit Erysiphe graminis (Mehltau) inkubiert. Nachdem 10 % der Blattoberfläche infiziert sind, werden die Schösslinge mit einer wässrigen Dispersion von Propiconazol oder Propiconazol und Carbendazin behandelt. Parallel dazu werden Dispersionen dieser beiden Wirkstoffe in Kombination mit Morpholin Arylsulfonaten wie auch mit dem jeweiligen Morpholin Arylsulfonat der Formel II allein behandelt. Nach 7, 14 und 21 Tagen wird der Mehltaubefall, die nicht vom Mehltau befallene Blattfläche sowie die Schädigung (phytotoxische Wirkung) der Versuchspflanze bonitiert. Die phytotoxische Wirkung wird in einem 9-stufigen Bonitierungsschema bewertet, in welchem 1 für keine Schädigung und 9 für eine totale Schädigung der Versuchspflanze steht. Der Mehltaubefall sowie die Bonitierung der nicht befallenen Pflanzenteile werden in % der jeweils infizierten bzw. nicht befallenen Blattflächen angegeben.

Tabelle 1

| Bei-spiel | | g/ha | 7 Tage A | 7 Tage B | 7 Tage B | 14 Tage A | 14 Tage B | 14 Tage C | 21 Tage A | 21 Tage B | 21 Tage C |
|---|---|---|---|---|---|---|---|---|---|---|---|
| - | Unbehandelte Kontrolle | - | 25.4 | 1.8 | - | 88.0 | 7.6 | 14.6 | 92.6 | 8.6 | 8.2 |
| - | 2,6-Dimethylmorpholin-cumolsulfonate (DMMCS) | 480 | 29.4 | 2.4 | - | 86.4 | 7.8 | 15.5 | 96.0 | 8.5 | 4.6 |
| - | Morpholincumol-sulfonat (MCS) | 480 | 32.6 | 2.2 | - | 89.0 | 7.9 | 12.0 | 97.6 | 8.9 | 3.4 |
| - | Propiconazol | 125 | 17.2 | 1.4 | - | 32.6 | 3.6 | 72.4 | 71.6 | 6.0 | 33.4 |
| 6 | Propiconazol + DMMCS | 125 + 480 | 14.8 | 1.8 | - | 14.8 | 2.6 | 83.0 | 35.4 | 4.2 | 68.0 |
| - | Propiconazol + Carbendazim | 125 + 100 | 14.6 | 1.8 | - | 27.2 | 3.4 | 72.6 | 51.0 | 5.6 | 45.0 |
| 7 | Propiconazol + Carbendazim + DMMCS | 125 + 100 + 480 | 16.6 | 2.0 | - | 19.2 | 2.6 | 83.6 | 28.0 | 4.3 | 71.0 |

A = mit Mehltau befallene Blattfläche (in %)

B = Phytotoxische Wirkung

C = Unbefallene Blattfläche (in %)

Beispiele 8 bis 11: Feldversuche

In mehrfach wiederholten Feldversuchen wird die Kontrolle von Mehltau bei Frühgerstensorte "Golden Promise" unter Verwendung verschiedener mikrobizider Mittel verglichen. In den Versuchen wird 27 Tage

nach der ersten Applikation in der jeweils angegebenen Aufwandmenge die Behandlung wiederholt und 37 Tage nach der ersten Applikation erneut bonitiert. Die Ergebnisse sind in Spalte A und Spalte B der Tabelle 2 wiedergegeben. Spalte C betrifft die im Feldversuch ermittelten Erntemengen.

Tabelle 2

| Beispiel | | g/ha | A | B | C |
|---|---|---|---|---|---|
| -<br>- | Unbehandelte Kontrollmenge<br>Propiconazol | <br>125 | 22.4<br>5.5 | 74.0<br>27.6 | 4.88<br>5.19 |
| - | Propiconazol<br>Carbendazim | 125)<br>100) | 8.76 | 36.8 | 5.31 |
| 8 | Propiconazol<br>MCS | 125)<br>480) | 3.8 | 19.4 | 5.41 |
| 9 | Propiconazol<br>DMMCS | 125)<br>480) | 3.7 | 23.2 | 5.26 |
| 10 | Propiconazol<br>Carbendazim<br>MCS | 125)<br>100)<br>480) | 7.90 | 29.0 | 5.51 |
| 11 | Propiconazol<br>Carbendazim<br>DMMCS | 125)<br>100)<br>480) | 5.80 | 23.4 | 5.45 |

A = Mehltaubefall in % 27 Tage nach 1. Applikation
B = Mehltaubefall in % 27 Tage nach 1. und 10 Tage nach 2. Applikation
C = Geerntete Getreidemenge in [t/ha]

**Patentansprüche**
**Patentansprüche für folgende Vertragsstaaten : BE, CH, DE, FR, GB, IT, LI, LU, NL, SE**

**1.** Mikrobizides Mittel bestehend aus eine Verbindung der Formel I

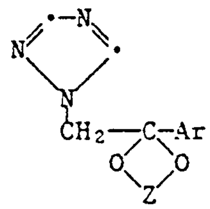

I

oder eines Säureadditionssalzes dieser Verbindung;
worin
Z $-CH_2CH_2-$, $-CH_2CH_2CH_2-$, $-CH(CH_3)-CH(CH_3)-$ oder $-CH_2-CH(C_1-C_{10}\text{-alkyl})-$; und
Ar einen Thiophenyl-, Halogenthiophenyl-, Naphthalinyl- oder einen unsubstituierten oder gegebenenfalls bis zu dreifach durch Halogen, $C_1$-$C_4$-Alkyl, $C_1$-$C_4$-Alkoxy, Cyano oder Nitro substituierten Phenylrest
bedeutet.

und eine synergistisch wirksame Menge eines Salzes der Formel II

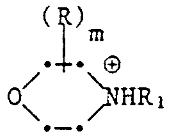 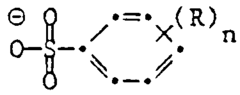 II,

in welcher
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet.

**2.** Mikrobizides Mittel gemäss Anspruch 1, enthaltend als Verbindung der Formel I
1-[2-(2,4-Dichlorophenyl)-1,3-dioxolan-2-ylmethyl]-1H-1,2,4-triazol;
1-[2-(2,4-Dichlorophenyl)-4-methyl-1,3-dioxolan-2-ylmethyl]-1H-1,2,4-triazol;
1-[2-(2,4-Dichlorophenyl)-4-ethyl-1,3-dioxolan-2-ylmethyl]-1H-1,2,4-triazol;
1-[2-(2,4-Dichlorophenyl)-4-propyl-1,3-dioxolan-2-ylmethyl]-1H-1,2,4-triazol (Propiconazol); oder
1-[2-(2,4-Dichlorophenyl)-4-pentyl-1,3-dioxolan-2-ylmethyl]-1H-1,2,4-triazol.

**3.** Mikrobizides Mittel gemäss einem der Ansprüche 1 oder 2, enthaltend als Verbindung der Formel II
Morpholin Benzolsulfonat,
Morpholin Toluolsulfonat,
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Xylolsulfonat,
2,6-Dimethylmorpholin Benzolsulfonat,
2,6-Dimethylmorpholin Xylolsulfonat,
2,2,6-Trimethylmorpholin Cumolsulfonat oder
2,2,6,6-Tetramethylmorpholin Cumolsulfonat.

**4.** Mittel gemäss einem der Ansprüche 1 bis 3, enthaltend weitere Hilfs- und Trägerstoffe.

**5.** Mittel gemäss einem der Ansprüche 1 bis 4, enthaltend mindestens eine weitere agrochemisch wirksame Substanz.

**6.** Mittel gemäss Anspruch 5, enthaltend als weitere agrochemisch wirksame Substanz:
a) ein Düngemittel, Spurenelemente oder andere Mittel, welche das Pflanzenwachstum beeinflussen,
b) ein selektives Herbizid,
c) ein Fungizid,
d) ein Bakterizid,
e) ein Nematozid oder
f) ein Molluskizid.

**7.** Mittel gemäss Anspruch 6, enthaltend Tridemorph und/oder Carbendazim als weiteres Fungizid.

**8.** Lösungskonzentrat gemäss einem der Ansprüche 1 bis 7, enthaltend bis an 75 Gew.-%/Vol.-% einer Verbindung der Formel I gelöst in einer bis zu 70 Gew.-%/Vol.-% wässrigen Lösung eines Salzes der Formel II, mit der Haßgabe, daß im Formel II $R_1$ nicht Wasserstoff ist, wenn m gleich 0 ist.

**9.** Verfahren zur Behandlung von durch mikrobielle Infektionen verursachte Pflanzenkrankheiten, dadurch gekennzeichnet, dass man ein Mittel gemäss einem der Ansprüche 1 bis 8 auf die Pflanze, auf Pflanzenteile, auf das Vermehrungsgut der Pflanzen oder auf den Lebensraum der Pflanzen einwirken lässt.

**10.** Verfahren gemäss Anspruch 9 zur Behandlung von Kulturpflanzen.

**11.** Verfahren gemäss Anspruch 10 zur Behandlung von Getreide.

**12.** Verfahren gemäss einem der Ansprüche 9 bis 11, dadurch gekennzeichnet, dass man das Mittel in einer Aufwandmenge von 50 g bis 5 kg Aktivsubstanz je Hektar anwendet.

**13.** Salze der Formel II

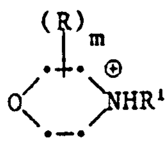 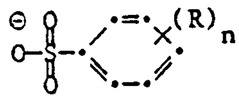 II,

worin
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4

bedeutet, mit der Massgabe, dass

**14.** Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Cumolsulfonat,
2,6-Dimethylmorpholin Cumolsulfonat,
2,2,6-Trimethylmorpholin Cumolsulfonat,
2,2,6,6-Tetramethylmorpholin Cumolsulfonat
gemäss Anspruch 15.

**15.** Verfahren zur Herstellung von Salzen der Formel II gemäss Anspruch, 13 oder 14, dadurch gekennzeichnet, dass man ein Morpolin der Formel III,

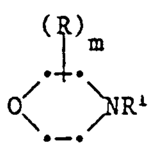 III,

$R^1$, R und m wie in Anspruch 13 definiert sind, mit einer Sulfonsäure der Formel IV

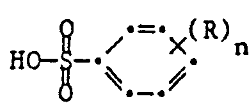 IV,

in der
R unabhängig voneinander $C_1$-$C_4$-Alkyl, und
n 0, 1, 2, 3 oder 4 bedeutet,
umsetzt.

**16.** Verfahren zur Herstellung eines Mittels gemäss einem der Ansprüche 1 bis 8, dadurch gekennzeichnet,

dass man die Aktivsubstanzen der Formel I und II mit den Hilfs- und Trägerstoffen sowie gewünschtenfalls mit den weiteren Aktivsubstanzen und Lösungsmitteln in inniger Weise vermischt, mit der Haßgabe, daß das losungsmittel nicht Wasser ist, wenn im Formel II $R_1$ für Wasserstoff und m gleich 0 stehen.

**17.** Verfahren zur Herstellung eines Lösungskonzentrats gemäss Anspruch 8, dadurch gekennzeichnet, dass man eine Verbindung der Formel I in einer wässrigen Lösung eines Salzes der Formel II löst.

**18.** Vermehrungsgut von Kulturpflanzen, dadurch gekennzeichnet, dass es mit einem Mittel gemäss einem der Ansprüche 1 bis 8 behandelt worden ist.

**19.** Mittel gemäss einem der Ansprüche 1 bis 8, enthaltend Propiconazol als Verbindung der Formel I.

**Patentansprüche für folgende Vertragsstaaten : AT, GR**

**1.** Mikrobizides Mittel bestehend aus einem Verbindung der Formel I

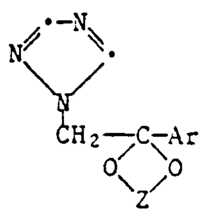

I

oder eines Säureadditionssalzes dieser Verbindung;
worin
Z $-CH_2CH_2-$, $-CH_2CH_2CH_2-$, $-CH(CH_3)-CH(CH_3)-$ oder $-CH_2-CH(C_1-C_{10}\text{-alkyl})-$; und
Ar einen Thiophenyl-, Halogenthiophenyl-, Naphthalinyl- oder einen unsubstituierten oder gegebenenfalls bis zu dreifach durch Halogen, $C_1$-$C_4$-Alkyl, $C_1$-$C_4$-Alkoxy, Cyano oder Nitro substituierten Phenylrest
bedeutet.

und eine synergistisch wirksame Menge eines Salzes der Formel II

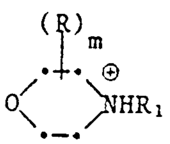

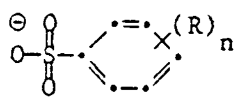

II,

in welcher
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet.

**2.** Mikrobizides Mittel gemäss Anspruch 1, enthaltend als Verbindung der Formel I
1-[2-(2,4-Dichlorophenyl)-1,3-dioxolan-2-ylmethyl]-1H-1,2,4-triazol;
1-[2-(2,4-Dichlorophenyl)-4-methyl-1,3-dioxolan-2-ylmethyl]-1H-1,2,4-triazol;
1-[2-(2,4-Dichlorophenyl)-4-ethyl-1,3-dioxolan-2-ylmethyl]-1H-1,2,4-triazol;
1-[2-(2,4-Dichlorophenyl)-4-propyl-1,3-dioxolan-2-ylmethyl]-1H-1,2,4-triazol (Propiconazol); oder
1-[2-(2,4-Dichlorophenyl)-4-pentyl-1,3-dioxolan-2-ylmethyl]-1H-1,2,4-triazol.

**3.** Mikrobizides Mittel gemäss einem der Ansprüche 1 oder 2, enthaltend als Verbindung der Formel II

Morpholin Benzolsulfonat,
Morpholin Toluolsulfonat,
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Xylolsulfonat,
2,6-Dimethylmorpholin Benzolsulfonat,
2,6-Dimethylmorpholin Xylolsulfonat,
2,2,6-Trimethylmorpholin Cumolsulfonat oder
2,2,6,6-Tetramethylmorpholin Cumolsulfonat.

**4.** Mittel gemäss einem der Ansprüche 1 bis 3, enthaltend weitere Hilfs- und Trägerstoffe.

**5.** Mittel gemäss einem der Ansprüche 1 bis 4, enthaltend mindestens eine weitere agrochemisch wirksame Substanz.

**6.** Mittel gemäss Anspruch 5, enthaltend als weitere agrochemisch wirksame Substanz:
a) ein Düngemittel, Spurenelemente oder andere Mittel, welche das Pflanzenwachstum beeinflussen,
b) ein selektives Herbizid,
c) ein Fungizid,
d) ein Bakterizid,
e) ein Nematozid oder
f) ein Molluskizid.

**7.** Mittel gemäss Anspruch 6, enthaltend Tridemorph und/oder Carbendazim als weiteres Fungizid.

**8.** Lösungskonzentrat gemäss einem der Ansprüche 1 bis 7, enthaltend bis an 75 Gew.-%/Vol.-% einer Verbindung der Formel I gelöst in einer bis zu 70 Gew.-%/Vol.-% wässrigen Lösung eines Salzes der Formel II, mit der Haßgabe dass in Formel II $R_1$ nicht Wasserstoff ist, wenn m gleich 0 ist.

**9.** Verfahren zur Behandlung von durch mikrobielle Infektionen verursachte Pflanzenkrankheiten, dadurch gekennzeichnet, dass man ein Mittel gemäss einem der Ansprüche 1 bis 8 auf die Pflanze, auf Pflanzenteile, auf das Vermehrungsgut der Pflanzen oder auf den Lebensraum der Pflanzen einwirken lässt.

**10.** Verfahren gemäss Anspruch 9 zur Behandlung von Kulturpflanzen.

**11.** Verfahren gemäss Anspruch 10 zur Behandlung von Getreide.

**12.** Verfahren gemäss einem der Ansprüche 9 bis 11, dadurch gekennzeichnet, dass man das Mittel in einer Aufwandmenge von 50 g bis 5 kg Aktivsubstanz je Hektar anwendet.

**13.** Verfahren zur Herstellung von Salzen der Formel II

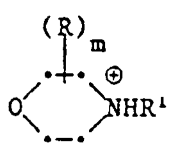 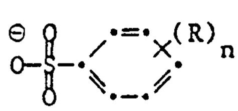 II

worin
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4

bedeutet, mit der Massgabe, dass
2,6-Dimethylmorpholin Benzolsulfonat
2,6-Dimethylmorpholin o-Toluolsulfonat
2,6-Dimethylmoholin m-Toluolsulfonat
2,6-Dimethylmorpholin p-Toluolsulfonat
2,6-Dimethylmorpholin 2,4,6-Trimethylbenzolsulfonat
N-Butylmorpholin Benzolsulfonat
N-Butylmorpholin o-Toluolsulfonat
N-Butylmorpholin m-Toluolsulfonat
N-Butylmorpholin p-Toluolsulfonat und
N-Butylmorpholin 2,4,6-Trimethylbenzolsulfonat nicht umfasst sind, dadurch gekennzeichnet, dass man ein Morpholin der Formel III

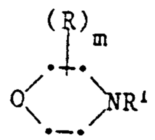

III,

in der
R unabhängig voneinander $C_1$-$C_4$-Alkyl,
$R^1$ Wasserstoff oder $C_1$-$C_4$-Alkyl und
m 0, 1, 2, 3 oder 4 bedeutet, mit einer Sulfonsäure der Formel IV

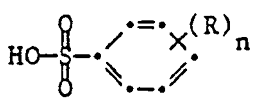

IV,

in der
R unabhängig voneinander $C_1$-$C_4$-Alkyl, und
n 0, 1, 2, 3 oder 4 bedeutet,
umsetzt.

**14.** Verfahren gemäss Anspruch 13 zur Herstellung von
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Cumolsulfonat,
2,6-Dimethylmorpholin Cumolsulfonat,
2,2,6-Trimethylmorpholin Cumolsulfonat,
2,2,6,6-Tetramethylmorpholin Cumolsulfonat.

**15.** Verfahren zur Herstellung eines Mittels gemäss einem der Ansprüche 1 bis 8, dadurch gekennzeichnet, dass man die Aktivsubstanzen der Formel I und II mit den Hilfs- und Trägerstoffen sowie gewünschtenfalls mit den weiteren Aktivsubstanzen und Lösungsmitteln in inniger Weise vermischt, mit der Maßgabe, dass das Lösungsmittel nicht Wasser ist, wenn in Formel II $R_1$ für Wasserstoff und m gleich 0 stehen.

**16.** Verfahren zur Herstellung eines Lösungskonzentrats gemäss Anspruch 8, dadurch gekennzeichnet, dass man eine Verbindung der Formel I in einer wässrigen Lösung eines Salzes der Formel II löst.

**17.** Mittel gemäss einem der Ansprüche 1 bis 8, enthaltend Propiconazol als Verbindung der Formel I.

**Patentansprüche für folgenden Vertragsstaat : ES**

1. Verfahren zur Herstellung eines mikrobiziden Mittels, bestehend aus einer synergistischen Mischung einer Verbindung der Formel I

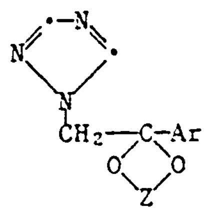

I

oder eines Säureadditionssalzes dieser Verbindung;
worin
Z $-CH_2CH_2-$, $-CH_2CH_2CH_2-$, $-CH(CH_3)-CH(CH_3)-$ oder $-CH_2-CH(C_1-C_{10}\text{-alkyl})-$; und
Ar einen Thiophenyl-, Halogenthiophenyl-, Naphthalinyl- oder einen unsubstituierten oder gegebenenfalls bis zu dreifach durch Halogen, $C_1$-$C_4$-Alkyl, $C_1$-$C_4$-Alkoxy, Cyano oder Nitro substituierten Phenylrest
bedeutet
und eine synergistisch wirksame Menge eines Salzes der Formel II

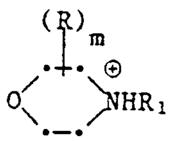

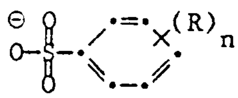

II,

in welcher
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet, dadurch gekennzeichnet, dass man die Aktivsubstanzen der Formel I und II mit Hilfsstoffen, Trägerstoffen und/oder Lösungsmittel in inniger Weise vermischt und/oder löst, mit der Maßgabe, dass das Lösungs mittel nicht Wasser ist, wenn in Formel II $R_1$ für Wasserstoff und m für 0 stehen.

2. Verfahren gemäss Anspruch 1, unter Verwendung von
1-[2-(2,4-Dichlorophenyl)-1,3-dioxolan-2-ylmethyl]-1H-1,2,4-triazol,
1-[2-(2,4-Dichlorophenyl)-4-methyl-1,3-dioxolan-2-ylmethyl]-1H-1,2,4-triazol,
1-[2-(2,4-Dichlorophenyl)-4-ethyl-1,3-dioxolan-2-ylmethyl]-1H-1,2,4-triazol,
1-[2-(2,4-Dichlorophenyl)-4-propyl-1,3-dioxolan-2-ylmethyl]-1H-1,2,4-triazol (Propiconazol) oder
1-[2-(2,4-Dichlorophenyl)-4-pentyl-1,3-dioxolan-2-ylmethyl]-1H-1,2,4-triazol
als Verbindung der Formel I.

3. Verfahren gemäss Anspruch 1 oder 2, unter Verwendung von
Morpholin Benzolsulfonat,
Morpholin Toluolsulfonat,
Morpholin Cumolsulfonat,
N-Methylmorpholin Benzolsulfonat,
N-Methylmorpholin Cumolsulfonat,
2-Methylmorphoiin Benzolsulfonat,
2-Methylmorpholin Toluolsulfonat,
2-Methylmorphoiin Xylolsulfonat,
2,6-Dimethylmorpholin Benzolsulfonat,
2,6-Dimethylmorpholin Xylolsulfonat,

2,2,6-Trimethylmorpholin Cumolsulfonat oder
2,2,6,6-Tetramethylmorpholin Cumolsulfonat
als Verbindung der Formel II.

4. Verfahren gemäss einem der Ansprüche 1 bis 3, dadurch gekennzeichnet, dass man neben den Aktivsubstanzen I und II mindestens eine weitere agrochemisch wirksame Substanz in inniger Weise mit den Hilfsstoffen, Trägerstoffen und/oder Lösungsmitteln vermischt und/oder löst.

5. Verfahren gemäss Anspruch 4, dadurch gekennzeichnet, dass man als weitere agrochemische Substanz
   a) ein Düngemittel, Spurenelemente oder andere Mittel, welche das Pflanzenwachstum beeinflussen,
   b) ein selektives Herbizid,
   c) ein Fungizid,
   d) ein Bakterizid,
   e) ein Nematozid oder
   f) ein Molluskizid

   verwendet.

6. Verfahren gemäss Anspruch 5, dadurch gekennzeichnet, dass man Tridemorph und/oder Carbendazim als weiteres Fungizid verwendet.

7. Verfahren gemäss einem der Ansprüche 1 bis 6, dadurch gekennzeichnet, dass man bis an 75 Gew.-%`/Vol.-% einer Verbindung der Formel I in einer bis zu 70 Gew.-%/Vol.-% wässrigen Lösung eines Salzes der Formel II löst.

8. Verfahren zur Behandlung von durch mikrobielle Infektionen verursachte Pflanzenkrankheiten, dadurch gekennzeichnet, dass man ein, nach dem Verfahren gemäss einem der Ansprüche 1 bis 7 erhältliches Mittel auf die Pflanze, auf Pflanzenteile, auf das Vermehrungsgut der Pflanzen oder auf den Lebensraum der Pflanzen einwirken lässt.

9. Verfahren gemäss Anspruch 8 zur Behandlung von Kulturpflanzen.

10. Verfahren gemäss Anspruch 9 zur Behandlung von Getreide.

11. Verfahren gemäss einem der Ansprüche 8 bis 10, dadurch gekennzeichnet, dass man das mikrobizide Mittel in einer Aufwandmenge von 50 g bis 5 kg Aktivsubstanz je Hektar anwendet.

12. Verfahren zur Herstellung von Salzen der Formel II

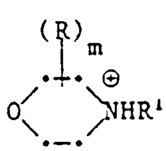 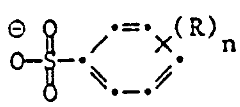 II,

worin
R unabhängig voneinander $C_1$-$C_4$-Alkyl,
$R^1$ Wasserstoff oder $C_1$-$C_4$-Alkyl,
m 0, 1, 2, 3 oder 4 und
n 0, 1, 2, 3 oder 4
bedeutet, mit der Massgabe, dass
2,6-Dimethylmorpholin Benzolsulfonat
2,6-Dimethylmorpholin o-Toluolsulfonat
2,6-Dimethylmorpholin m-Toluolsulfonat
2,6-Dimethylmorpholin p-Toluolsulfonat
2,6-Dimethylmorpholin 2,4,6-Trimethylbenzolsulfonat
N-Butylmorpholin Benzolsulfonat

N-Butylmorpholin o-Toluolsulfonat
N-Butylmorpholin m-Toluolsulfonat
N-Butylmorpholin p-Toluolsulfonat und
N-Butylmorpholin 2,4,6-Trimethylbenzolsulfonat nicht umfasst sind, dadurch gekennzeichnet, dass man ein Morpholin der Formel III

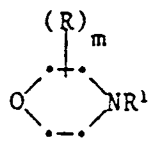 III,

in der

die Reste $R^1$, R und m wie zuvor definiert sind, mit Sulfonsäure der Formel IV

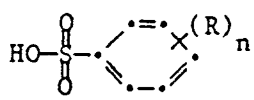 IV,

in der

in der R und n wie zuvor definiert ist, umsetzt.

**13.** Verfahren gemäss Anspruch 12 zur Herstellung von
Morpholin Cumolsulfonat,
N-Methylmorpholin Benzolsulfonat,
N-Methylmorpholin Cumolsulfonat,
2-Methylmorpholin Benzolsulfonat,
2-Methylmorpholin Toluolsulfonat,
2-Methylmorpholin Cumolsulfonat,
2,6-Dimethylmorpholin Cumolsulfonat,
2,2,6-Trimethylmorpholin Cumolsulfonat,
2,2,6,6-Tetramethylmorpholin Cumolsulfonat.

**Claims**
**Claims for the following Contracting States : BE, CH, DE, FR, GB, IT, LI, LU, NL, SE**

**1.** A microbicidal composition comprising a compound of formula I

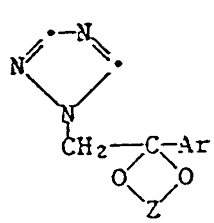 (I)

or an acid addition salt thereof;
in which
Z is $-CH_2CH_2-$, $-CH_2CH_2CH_2-$, $-CH(CH_3)-CH(CH_3)-$ or $-CH_2-CH(C_1-C_{10}alkyl)-$; and
Ar is a thiophenyl, halothiophenyl or naphthalinyl radical, or a phenyl radical that is unsubstituted or substituted by up to 3 halogen, $C_1-C_4$alkyl, $C_1-C_4$alkoxy, cyano or nitro substituents,

and a synergistically effective amount of a salt of formula II

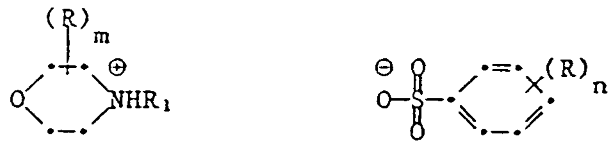 (II)

in which
each R, independently of the other(s), is $C_1$-$C_4$ alkyl,
$R^1$ is hydrogen or $C_1$-$C_4$ alkyl,
m is 0, 1, 2, 3 or 4 and
n is 0, 1, 2, 3 or 4.

2. A microbicidal composition according to claim 1, comprising as compound of formula I
1-[2-(2,4-Dichlorophenyl)-1,3-dioxolan-2-ylmethyl]-1H-1,2,4-triazole;
1-[2-(2,4-dichlorophenyl)-4-methyl-1,3-dioxolan-2-ylmethyl]-1H-1,2,4-triazole;
1-[2-(2,4-dichlorophenyl)-4-ethyl-1,3-dioxolan-2-yl-methyl]-1H-1,2,4-triazole;
1-[2-(2,4-dichlorophenyl)-4-propyl-1,3-dioxolan-2-ylmethyl]-1H-1,2,4-triazole (propiconazole); or
1-[2-(2,4-dichlorophenyl)-4-pentyl-1,3-dioxolan-2-ylmethyl]-1H-1,2,4-triazole.

3. A microbicidal composition according to claim 1 or 2, comprising as compound of formula II
morpholine benzene sulfonate,
morpholine toluene sulfonate,
morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine xylene sulfonate,
2,6-dimethylmorpholine benzene sulfonate,
2,6-dimethylmorpholine xylene sulfonate,
2,2,6-trimethylmorpholine cumene sulfonate or
2,2,6,6-tetramethylmorpholine cumene sulfonate.

4. A composition according to any one of claims 1 to 3 comprising further adjuvants and carriers.

5. A composition according to any one of claims 1 to 4 comprising at least one further agrochemically active product.

6. A composition according to claim 5 comprising as the further agrochemically active product
a) a fertilizer, micronutrient donors or other preparations that influence plant growth,
b) a selective herbicide,
c) a fungicide,
d) a bactericide,
e) a nematicide, or
f) a molluscicide.

7. A composition according to claim 6 comprising as the further fungicide tridemorph and/or carbendazim.

8. A solution concentrate according to any one of claims 1 to 7 comprising up to 75 % by weight/% by volume of a compound of formula I dissolved in an up to 70 % by weight/% by volume aqueous solution of a salt of formula II, with the proviso that in formula II $R_1$ is not hydrogen when m is 0.

9. A method of treating plant diseases caused by microbial infections, which comprises applying to the plant, parts of the plant, to the propagating material or to the locus of the plant a composition as claimed in any one of claims 1 to 8.

**10.** A method according to claim 9 for the treatment of cultivated plants.

**11.** A method according to claim 10 for the treatment of cereals.

**12.** A method according to any one of claims 9 to 11 wherein the composition is applied at a rate of application of from 50 g to 5 kg of active ingredient per hectare.

**13.** Salts of formula II

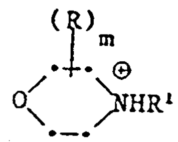 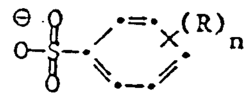 II

in which
each R, independently of the other(s), is $C_1$-$C_4$alkyl,
$R^1$ is hydrogen or $C_1$-$C_4$alkyl,
m is 0, 1, 2, 3 or 4 and
n is 0, 1, 2, 3 or 4,
with the proviso that
2,6-dimethylmorpholine benzene sulfonate,
2,6-dimethylmorpholine o-toluene sulfonate,
2,6-dimethylmorpholine m-toluene sulfonate,
2,6-dimethylmorpholine p-toluene sulfonate,
2,6-dimethylmorpholine 2,4,6-trimethylbenzene sulfonate,
N-butylmorpholine benzene sulfonate,
N-butylmorpholine o-toluene sulfonate,
N-butylmorpholine m-toluene sulfonate,
N-butylmorpholine p-toluene sulfonate and
N-butylmorpholine 2,4,6-trimethylbenzene sulfonate are
not included.

**14.** Morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine cumene sulfonate,
2,6-dimethylmorpholine cumene sulfonate,
2,2,6-trimethylmorpholine cumene sulfonate,
2,2,6,6-tetramethylmorpholine cumene sulfonate
according to claim 15.

**15.** A process for the preparation of a salt of formula II according to claim 13 or 14, which process comprises reacting a morpholine of formula III

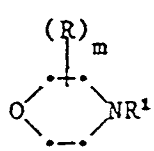 III

in which $R^1$, R and m are as defined in claim 13, with a sulfonic acid of formula IV

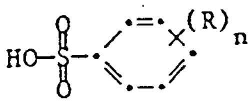

IV,

in which
each R, independently of the other(s), is $C_1$-$C_4$alkyl and
n is 0, 1, 2, 3 or 4.

**16.** A process for the preparation of a composition according to any one of claims 1 to 8, which process comprises intimately mixing the active compounds of formulae I and II with adjuvants and carriers and, if desired, with further active products and solvents, with the proviso that the solvent is not water when in formula II $R_1$ is hydrogen and m is 0.

**17.** A process for the preparation of a solution concentrate according to claim 8, which process comprises dissolving a compound of formula I in an aqueous solution of a salt of formula II.

**18.** Propagating material of cultivated plants which has been treated with a composition according to any one of claims 1 to 8.

**19.** A composition according to any one of claims 1 to 8 comprising propiconazole as compound of formula I.

**Claims for the following Contracting States : AT, GR**

**1.** A microbicidal composition comprising a compound of formula I

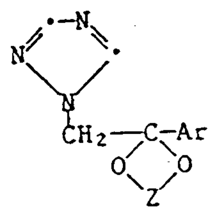

(I)

or an acid addition salt thereof;
in which
Z is $-CH_2CH_2-$, $-CH_2CH_2CH_2-$, $-CH(CH_3)-CH(CH_3)-$ or
$-CH_2-CH(C_1-C_{10}alkyl)-$; and
Ar is a thiophenyl, halothiophenyl or naphthalinyl radical, or a phenyl radical that is unsubstituted or substituted by up to 3 halogen, $C_1$-$C_4$alkyl, $C_1$-$C_4$alkoxy, cyano or nitro substituents,

and a synergistically effective amount of a salt of formula II

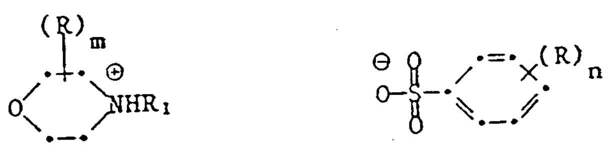

(II)

in which
each R, independently of the other(s), is $C_1$-$C_4$alkyl,
$R^1$ is hydrogen or $C_1$-$C_4$alkyl,
m is 0, 1, 2, 3 or 4 and
n is 0, 1, 2, 3 or 4.

**2.** A microbicidal composition according to claim 1, comprising as compound of formula I
1-[2-(2,4-dichlorophenyl)-1,3-dioxolan-2-ylmethyl]-1H-1,2,4-triazole;
1-[2-(2,4-dichlorophenyl)-4-methyl-1,3-dioxolan-2-ylmethyl]-1H-1,2,4-triazole;
1-[2-(2,4-dichlorophenyl)-4-ethyl-1,3-dioxolan-2-yl-methyl]-1H-1,2,4-triazole;
1-[2-(2,4-dichlorophenyl)-4-propyl-1,3-dioxolan-2-ylmethyl]-1H-1,2,4-triazole (propiconazole); or
1-[2-(2,4-dichlorophenyl)-4-pentyl-1,3-dioxolan-2-ylmethyl]-1H-1,2,4-triazole.

**3.** A microbicidal composition according to claim 1 or 2, comprising as compound of formula II
morpholine benzene sulfonate,
morpholine toluene sulfonate,
morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine xylene sulfonate,
2,6-dimethylmorpholine benzene sulfonate,
2,6-dimethylmorpholine xylene sulfonate,
2,2,6-trimethylmorpholine cumene sulfonate or
2,2,6,6-tetramethylmorpholine cumene sulfonate.

**4.** A composition according to any one of claims 1 to 3 comprising further adjuvants and carriers.

**5.** A composition according to any one of claims 1 to 4 comprising at least one further agrochemically active product.

**6.** A composition according to claim 5 comprising as the further agrochemically active product
a) a fertilizer, micronutrient donors or other preparations that influence plant growth,
b) a selective herbicide,
c) a fungicide,
d) a bactericide,
e) a nematicide, or
f) a molluscicide.

**7.** A composition according to claim 6 comprising as the further fungicide tridemorph and/or carbendazim.

**8.** A solution concentrate according to any one of claims 1 to 7 comprising up to 75 % by weight/% by volume of a compound of formula I dissolved in an up to 70 % by weight/% by volume aqueous solution of a salt of formula II, with the proviso that in formula II $R_1$ is not hydrogen when $\underline{m}$ is 0.

**9.** A method of treating plant diseases caused by microbial infections, which comprises applying to the plant, parts of the plant, to the propagating material or to the locus of the plant a composition as claimed in any one of claims 1 to 8.

**10.** A method according to claim 9 for the treatment of cultivated plants.

**11.** A method according to claim 10 for the treatment of cereals.

**12.** A method according to any one of claims 9 to 11 wherein the composition is applied at a rate of application of from 50 g to 5 kg of active ingredient per hectare.

**13.** A process for the preparation of a salt of formula II

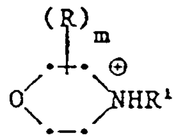 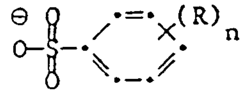 II

in which
each R, independently of the other(s), is $C_1$-$C_4$ alkyl,
$R^1$ is hydrogen or $C_1$-$C_4$ alkyl,
m is 0, 1, 2, 3 or 4 and
n is 0, 1, 2, 3 or 4,
with the proviso that
2,6-dimethylmorpholine benzene sulfonate,
2,6-dimethylmorpholine o-toluene sulfonate,
2,6-dimethylmorpholine m-toluene sulfonate,
2,6-dimethylmorpholine p-toluene sulfonate,
2,6-dimethylmorpholine 2,4,6-trimethylbenzene sulfonate,
N-butylmorpholine benzene sulfonate,
N-butylmorpholine o-toluene sulfonate,
N-butylmorpholine m-toluene sulfonate,
N-butylmorpholine p-toluene sulfonate and
N-butylmorpholine 2,4,6-trimethylbenzene sulfonate are not included, which process comprises reacting a morpholine of formula III

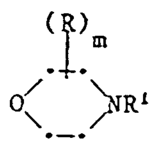 III

in which
each R, independently of the other(s), is $C_1$-$C_4$ alkyl,
$R^1$ is hydrogen or $C_1$-$C_4$ alkyl and
m is 0, 1, 2, 3 or 4,
with a sulfonic acid of formula IV

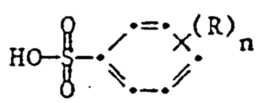 IV,

in which
each R, independently of the other(s), is $C_1$-$C_4$ alkyl and
n is 0, 1, 2, 3 or 4.

**14.** A process according to claim 13 for the preparation of
morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine cumene sulfonate,
2,6-dimethylmorpholine cumene sulfonate,
2,2,6-trimethylmorpholine cumene sulfonate and
2,2,6,6-tetramethylmorpholine cumene sulfonate.

**15.** A process for the preparation of a composition according to any one of claims 1 to 8, which process comprises intimately mixing the active compounds of formulae I and II with adjuvants and carriers and, if desired, with further active products and solvents, with the proviso that the solvent is not water when in formula II $R_1$ is hydrogen and <u>m</u> is 0.

**16.** A process for the preparation of a solution concentrate according to claim 8, which process comprises dissolving a compound of formula I in an aqueous solution of a salt of formula II.

**17.** A composition according to any one of claims 1 to 8 comprising propiconazole as compound of formula I.

**Claims for the following Contracting State : ES**

**1.** A process for the preparation of a microbicidal composition comprising a synergistic mixture of a compound of formula I

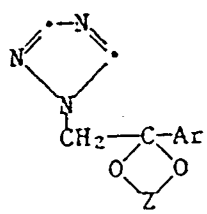 (I)

or of an acid addition salt thereof;
in which
Z is $-CH_2CH_2-$, $-CH_2CH_2CH_2-$, $-CH(CH_3)-CH(CH_3)-$ or $-CH_2-CH(C_1-C_{10}alkyl)-$; and
Ar is a thiophenyl, halothiophenyl or naphthalinyl radical, or a phenyl radical that is unsubstituted or substituted by up to 3 halogen, $C_1-C_4$alkyl, $C_1-C_4$alkoxy, cyano or nitro substituents,

and a synergistically effective amount of a salt of formula II

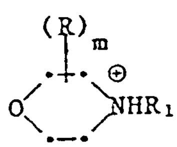 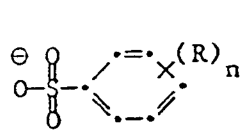 (II)

in which
each R, independently of the other(s), is $C_1-C_4$alkyl,
$R^1$ is hydrogen or $C_1-C_4$alkyl,
<u>m</u> is 0, 1, 2, 3 or 4 and
<u>n</u> is 0, 1, 2, 3 or 4,
which process comprises intimately mixing and/or dissolving the active compounds of formulae I and II with adjuvants, carriers and/or solvents, with the proviso that the solvent is not water when in formula II $R_1$ is hydrogen and <u>m</u> is 0.

**2.** A process according to claim 1, using as compound of formula I
1-[2-(2,4-dichlorophenyl)-1,3-dioxolan-2-ylmethyl]-1H-1,2,4-triazole,
1-(2-(2,4-dichlorophenyl)-4-methyl-1,3-dioxolan-2-ylmethyl]-1H-1,2,4-triazole,
1-[2-(2,4-dichlorophenyl)-4-ethyl-1,3-dioxolan-2-yl-methyl]-1H-1,2,4-triazole,
1-[2-(2,4-dichlorophenyl)-4-propyl-1,3-dioxolan-2-ylmethyl]-1H-1,2,4-triazole (propiconazole), or
1-[2-(2,4-dichlorophenyl)-4-pentyl-1,3-dioxolan-2-ylmethyl]-1H-1,2,4-triazole.

**3.** A process according to claim 1 or 2 using as compound of formula II

morpholine benzene sulfonate,
morpholine toluene sulfonate,
morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine xylene sulfonate,
2,6-dimethylmorpholine benzene sulfonate,
2,6-dimethylmorpholine xylene sulfonate,
2,2,6-trimethylmorpholine cumene sulfonate or
2,2,6,6-tetramethylmorpholine cumene sulfonate.

**4.** A process according to any one of claims 1 to 3 which comprises intimately mixing and/or dissolving with the adjuvants, carriers and/or solvents at least one further agrochemically active product besides the active compounds I and II.

**5.** A process according to claim 4 which comprises using as the further agrochemical product
a) a fertilizer, micronutrient donors or other preparations that influence plant growth,
b) a selective herbicide,
c) a fungicide,
d) a bactericide,
e) a nematicide, or
f) a molluscicide.

**6.** A process according to claim 5 which comprises using as the further fungicide tridemorph and/or carbendazim.

**7.** A process according to any one of claims 1 to 6 which comprises dissolving up to 75 % by weight/% by volume of a compound of formula I in an up to 70 % by weight/% by volume aqueous solution of a salt of formula II.

**8.** A method of treating plant diseases caused by microbial infections, which comprises applying to the plant, parts of the plant, to the propagating material or to the locus of the plant a composition obtainable in accordance with the process as claimed in any one of claims 1 to 7.

**9.** A method according to claim 8 for the treatment of cultivated plants.

**10.** A method according to claim 9 for the treatment of cereals.

**11.** A method according to any one of claims 8 to 10 wherein the microbicidal composition is applied at a rate of application of from 50 g to 5 kg of active ingredient per hectare.

**12.** A process for the preparation of salts of formula II

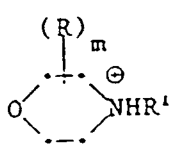
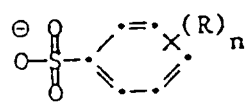

II

in which
each R, independently of the other(s), is $C_1$-$C_4$alkyl,
$R^1$ is hydrogen or $C_1$-$C_4$alkyl,
m is 0, 1, 2, 3 or 4 and
n is 0, 1, 2, 3 or 4,
with the proviso that

2,6-dimethylmorpholine benzene sulfonate,
2,6-dimethylmorpholine o-toluene sulfonate,
2,6-dimethylmorpholine m-toluene sulfonate,
2,6-dimethylmorpholine p-toluene sulfonate,
2,6-dimethylmorpholine 2,4,6-trimethylbenzene sulfonate,
N-butylmorpholine benzene sulfonate,
N-butylmorpholine o-toluene sulfonate,
N-butylmorpholine m-toluene sulfonate,
N-butylmorpholine p-toluene sulfonate and
N-butylmorpholine 2,4,6-trimethylbenzene sulfonate are not included, which process comprises reacting a morpholine of formula III

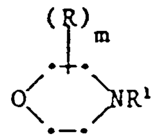

III

in which the radicals $R^1$, R and m are as defined above with a sulfonic acid of formula IV

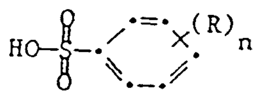

IV,

in which R and n are as defined above.

**13.** A process according to claim 12 for the preparation of
morpholine cumene sulfonate,
N-methylmorpholine benzene sulfonate,
N-methylmorpholine cumene sulfonate,
2-methylmorpholine benzene sulfonate,
2-methylmorpholine toluene sulfonate,
2-methylmorpholine cumene sulfonate,
2,6-dimethylmorpholine cumene sulfonate,
2,2,6-trimethylmorpholine cumene sulfonate,
2,2,6,6-tetramethylmorpholine cumene sulfonate.

## Revendications
**Revendications pour les Etats contractants suivants : BE, CH, DE, FR, GB, IT, LI, LU, NL, SE**

**1.** Produit microbicide consistant en un composé de formule I

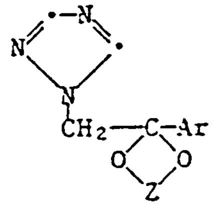

I

ou un sel de ce composé formé par addition avec un acide,
Z représentant $-CH_2CH_2-$, $-CH_2CH_2CH_2-$, $-CH(CH_3)-CH(CH_3)-$ou $-CH_2-CH$(alkyle en C 1-C 10)- et
Ar un groupe thiophényle, halogénothiophényle, naphtalényle ou un groupe phényle non substitué ou portant le cas échéant jusqu'à 3 substituants choisis parmi les halogènes, les groupes alkyle en C 1-C

4, alcoxy en C 1-C 4, cyano ou nitro, et une quantité synergétique efficace d'un sel de formule II

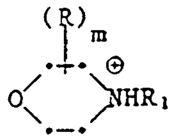 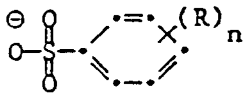 II,

dans laquelle
chacun des symboles R représente; indépendamment des autres, un groupe alkyle en C 1-C 4,
$R^1$ représente l'hydrogène ou un groupe alkyle en C 1-C 4,
m est égal à 0, 1, 2, 3 ou 4, et
n est égal à 0, 1, 2, 3 ou 4.

**2.** Produit microbicide selon la revendication 1, contenant en tant que composé de formule I :
le 1-[2-(2,4-dichlorophényl)-1,3-dioxolanne-2-ylméthyl]-1H-1,2,4-triazole,
le 1-[2-(2,4-dichlorophényl)-4-méthyl-1,3-dioxolanne-2-ylméthyl]-1H-1,2,4-triazole,
le 1-[2-(2,4-dichlorophényl)-4-éthyl-1,3-dioxolanne-2-ylméthyl]-1H-1,2,4-triazole,
le 1-[2-(2,4-dichlorophényl)-4-propyl-1,3-dioxolanne-2-ylméthyl]-1H-1,2,4-triazole, (Propiconazol); ou bien
le 1-[2-(2,4-dichlorophényl)-4-pentyl-1,3-dioxolanne-2-ylméthyl]-1H-1,2,4-triazole.

**3.** Produit microbicide selon l'une des revendications 1 ou 2, contenant en tant que composé de formule II :
le benzène-sulfonate de morpholine,
le toluène-sulfonate de morpholine,
le cumène-sulfonate de morpholine,
le benzène-sulfonate de N-méthylmorpholine,
le cumène-sulfonate de N-méthylmorpholine,
le benzène-sulfonate de 2-méthylmorpholine,
le toluène-sulfonate de 2-méthylmorpholine,
le xylène-sulfonate de 2-méthylmorpholine,
le benzène-sulfonate de 2,6-diméthylmorpholine,
le xylène-sulfonate de 2,6-diméthylmorpholine,
le cumène-sulfonate de 2,2,6-triméthylmorpholine, ou
le cumène-sulfonate de 2,2,6,6-tétraméthylmorpholine.

**4.** Produit selon une des revendications 1 à 3, contenant d'autres produits auxiliaires et véhicules.

**5.** Produit selon l'une des revendications 1 à 4, contenant au moins une autre substance possédant une activité agrochimique.

**6.** Produit selon revendication 5, contenant en tant qu'autre substance possédant une activité agrochimique :
a) un engrais, des oligo-éléments ou d'autres produits agissant sur la croissance des végétaux,
b) un herbicide sélectif,
c) un fongicide,
d) un bactéricide,
e) un nématocide ou
f) un mollusquicide.

**7.** Produit selon revendication 6, contenant du Tridemorph et/ou du Carbendazim en tant qu'autres fongicides.

**8.** Concentré en solution selon une des revendications 1 à 7, contenant jusqu'à 75 % en poids/volume d'un composé de formule I en solution dans une solution aqueuse d'un sel de formule II à une

concentration allant jusqu'à 70 % en poids/volume de ce dernier, sous réserve que, dans la formule II, $R^1$ ne peut représenter l'hydrogène lorsque m est égal à 0.

**9.** Procédé pour traiter des maladies des végétaux causées par des infections microbiennes, caractérisé en ce que l'on fait agir un produit selon une des revendications 1 à 8 sur la plante, sur des parties de la plante, sur les organes de reproduction de la plante ou sur l'habitat de la plante.

**10.** Procédé selon revendication 9, pour le traitement des végétaux cultivés.

**11.** Procédé selon la revendication 10, pour le traitement des céréales.

**12.** Procédé selon une des revendications 9 à 11, caractérisé en ce que l'on applique le produit à la dose de 50 g à 5 kg de substance active par ha.

**13.** Sels de formule II

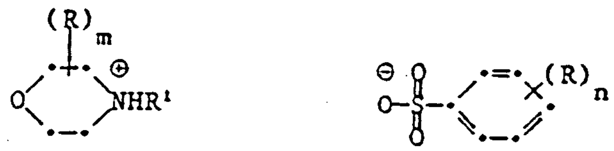

II,

dans laquelle
chacun des symboles R représente, indépendamment des autres, un groupe alkyle en C 1-C 4,
$R^1$ représente l'hydrogène ou un groupe alkyle en C 1-C 4,
m est égal à 0, 1, 2, 3 ou 4, et
n est égal à 0, 1, 2, 3 ou 4,
à l'exclusion des suivants :
le benzène-sulfonate de 2,6-diméthylmorpholine,
l'o-toluène-sulfonate de 2,6-diméthylmorpholine,
le m-toluène-sulfonate de 2,6-diméthylmorpholine,
le p-toluène-sulfonate de 2,6-diméthylmorpholine,
le 2,4,6-triméthylbenzène-sulfonate de 2,6-diméthylmorpholine,
le benzène-sulfonate de N-butylmorpholine,
l'o-toluène-sulfonate de N-butylmorpholine,
le m-toluène-sulfonate de N-butylmorpholine,
le p-toluène-sulfonate de N-butylmorpholine, et
le 2,4,6-triméthylbenzène-sulfonate de N-butylmorpholine.

**14.** Le cumène-sulfonate de morpholine,
le benzène-sulfonate de N-méthylmorpholine,
le cumène-sulfonate de N-méthylmorpholine,
le benzène-sulfonate de 2-méthylmorpholine,
le toluène-sulfonate de 2-méthylmorpholine,
le cumène-sulfonate de 2-méthylmorpholine,
le cumène-sulfonate de 2,6-diméthylmorpholine,
le cumène-sulfonate de 2,2,6-triméthylmorpholine,
le cumène-sulfonate de 2,2,6,6-tétraméthylmorpholine,
selon revendication 15.

**15.** Procédé de préparation des sels de formule II de la revendication 13 ou 14, caractérisé en ce que l'on fait réagir une morpholine de formule III

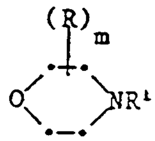

III,

dans laquelle $R^1$, R et m ont été définis dans la revendication 13, avec un acide sulfonique de formule IV

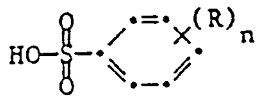

IV,

dans laquelle chacun des symboles R représente, indépendamment des autres, un groupe alkyle en C 1-C 4, et n est égal à 0, 1, 2, 3 ou 4.

**16.** Procédé de préparation d'un produit selon l'une des revendications 1 à 8, caractérisé en ce que l'on mélange intimement les substances actives de formules I et II avec les produits auxiliaires et véhicules et si on le désire les autres substances actives et les solvants, sous réserve que le solvant ne peut consister en l'eau lorsque, dans la formule II, $R^1$ représente l'hydrogène et m est égal à 0.

**17.** Procédé de préparation d'un concentré en solution selon revendication 8, caractérisé en ce que l'on dissout un composé de formule I dans une solution aqueuse d'un sel de formule II.

**18.** Organes de reproduction de végétaux cultivés, caractérisés en ce qu'ils ont été traités par un produit selon une des revendications 1 à 8.

**19.** Produit selon une des revendications 1 à 8, contenant du Propiconazol en tant que composé de formule I.

**Revendications pour les Etats contractants suivants : AT, GR**

**1.** Produit microbicide consistant en un composé de formule I

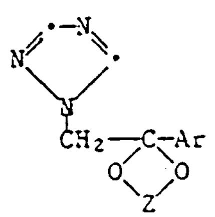

I

ou un sel de ce composé formé par addition avec un acide,
Z représentant $-CH_2CH_2-$, $-CH_2CH_2CH_2-$, $-CH(CH_3)-CH(CH_3)-$ou $-CH_2-CH$(alkyle en C 1-C 10)-; et
Ar un groupe thiophényle, halogénothiophényle, naphtalényle ou un groupe phényle non substitué ou portant éventuellement jusqu'à 3 substituants choisis parmi les halogènes, les groupes alkyle en C 1-C 4, alcoxy en C 1-C 4, cyano ou nitro,
et une quantité synergétique efficace d'un sel de formule II

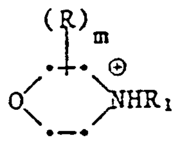 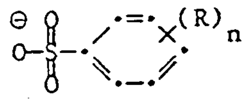 II,

dans laquelle
chacun des symboles R représente, indépendamment des autres, un groupe alkyle en C 1-C 4,
$R^1$ représente l'hydrogène ou un groupe alkyle en C 1-C 4,
m est égal à 0, 1, 2, 3 ou 4, et
n est égal à 0, 1, 2, 3 ou 4.

**2.** Produit microbicide selon revendication 1, contenant, en tant que composé de formule I :
le 1-[2-(2,4-dichlorophényl)-1,3-dioxolanne-2-ylméthyl]-1H-1,2,4-triazole,
le 1-[2-(2,4-dichlorophényl)-4-méthyl-1,3-dioxolanne-2-ylméthyl]-1H-1,2,4-triazole,
le 1-[2-(2,4-dichlorophényl)-4-éthyl-1,3-dioxolanne-2-ylméthyl]-1H-1,2,4-triazole,
le 1-[2-(2,4-dichlorophényl)-4-propyl-1,3-dioxolanne-2-ylméthyl]-1H-1,2,4-triazole (Propiconazol); et
le 1-[2-(2,4-dichlorophényl-4-pentyl-1,3-dioxolanne-2-ylméthyl]-1H-1,2,4-triazole.

**3.** Produit microbicide selon une des revendications 1 ou 2, contenant en tant que composé de formule II :
le benzène-sulfonate de morpholine,
le toluène-sulfonate de morpholine,
le cumène-sulfonate de morpholine,
le benzène-sulfonate de N-méthylmorpholine,
le cumène-sulfonate de N-méthylmorpholine,
le benzène-sulfonate de 2-méthylmorpholine,
le toluène-sulfonate de 2-méthylmorpholine,
le xylène-sulfonate de 2-méthylmorpholine,
le benzène-sulfonate de 2,6-diméthylmorpholine,
le xylène-sulfonate de 2,6-diméthylmorpholine,
le cumène-sulfonate de 2,2,6-triméthylmorpholine, ou
le cumène-sulfonate de 2,2,6,6-tétraméthylmorpholine.

**4.** Produit selon une des revendications 1 à 3, contenant d'autres produits auxiliaires et véhicules.

**5.** Produit selon une des revendications 1 à 4, contenant au moins une autre substance possédant une activité agrochimique.

**6.** Produit selon revendication 5, contenant en tant qu'autres substances possédant une activité agrochimique :
a) un engrais, des oligo-éléments ou d'autres produits agissant sur la croissance des végétaux;
b) un herbicide sélectif,
c) un fongicide,
d) un bactéricide,
e) un nématocide, ou bien
f) un mollusquicide.

**7.** Produit selon revendication 6, contenant du Tridemorph et/ou du Carbendazim en tant qu'autres fongicides.

**8.** Concentré en solution selon une des revendications 1 à 7, contenant jusqu'à 75 % en poids/volume d'un composé de formule I en solution dans une solution aqueuse d'un sel de formule II à une concentration allant jusqu'à 70 % en poids/volume, sous réserve que dans la formule II, $R^1$ ne peut représenter l'hydrogène lorsque m est égal à 0.

**9.** Procédé pour traiter les maladies des végétaux causées par des infections microbiennes, caractérisé

en ce que l'on fait agir un produit selon une des revendications 1 à 8 sur la plante, des parties de la plante, les organes de reproduction de la plante ou l'habitat de la plante.

**10.** Procédé selon revendication 9, pour le traitement des végétaux cultivés.

**11.** Procédé selon revendication 10, pour le traitement des céréales.

**12.** Procédé selon une des revendications 9 à 11, caractérisé en ce que l'on applique le produit à une dose de 50 g à 5 kg de substance active par ha.

**13.** Procédé de préparation des sels de formule II

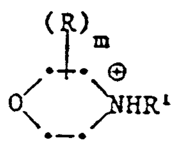 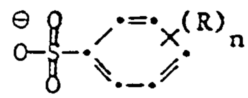 II

dans laquelle
chacun des symboles R représente, indépendamment des autres, un groupe alkyle en C 1-C 4,
$R^1$ représente l'hydrogène ou un groupe alkyle en C 1-C 4,
m est égal à 0, 1, 2, 3 ou 4, et
n est égal à 0, 1, 2, 3 ou 4,
à l'exclusion des sels suivants :
le benzène-sulfonate de 2,6-diméthylmorpholine,
l'o-toluène-sulfonate de 2,6-diméthylmorpholine,
le m-toluène-sulfonate de 2,6-diméthylmorpholine,
le p-toluène-sulfonate de 2,6-diméthylmorpholine,
le 2,4,6-triméthylbenzène-sulfonate de 2,6-diméthylmorpholine,
le benzène-sulfonate de N-butylmorpholine,
l'o-toluène-sulfonate de N-butylmorpholine,
le m-toluène-sulfonate de N-butylmorpholine,
le p-toluène-sulfonate de N-butylmorpholine, et
le 2,4,6-triméthylbenzène-sulfonate de N-butylmorpholine, caractérisé en ce que l'on fait réagir une morpholine de formule III

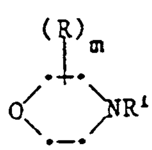 III,

dans laquelle
chacun des symboles R représente, indépendamment des autres, un groupe alkyle en C 1-C 4,
$R^1$ représente l'hydrogène ou un groupe alkyle en C 1-C 4, et
m est égal à 0, 1, 2, 3 ou 4,
avec un acide sulfonique de formule IV

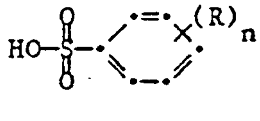 IV,

dans laquelle
chacun des symboles R représente, indépendamment des autres, un groupe alkyle en C 1-C 4, et

n est égal à 0, 1, 2, 3 ou 4.

**14.** Procédé selon revendication 13, pour la préparation
du cumène-sulfonate de morpholine,
du benzène-sulfonate de N-méthylmorpholine,
du cumène-sulfonate de N-méthylmorpholine,
du benzène-sulfonate de 2-méthylmorpholine,
du toluène-sulfonate de 2-méthylmorpholine,
du cumène-sulfonate de 2-méthylmorpholine,
du cumène-sulfonate de 2,6-diméthylmorpholine,
du cumène-sulfonate de 2,2,6-triméthylmorpholine,
du cumène-sulfonate de 2,2,6,6-tétraméthylmorpholine.

**15.** Procédé de préparation d'un produit selon une des revendications 1 à 8, caractérisé en ce que l'on mélange intimement les substances actives de formules I et II avec les produits auxiliaires et véhicules et si on le désire les autres substances actives et les solvants, sous réserve que le solvant ne peut consister en eau lorsque, dans la formule II, $R^1$ représente l'hydrogène et m est égal à 0.

**16.** Procédé de préparation d'un concentré en solution selon revendication 8, caractérisé en ce que l'on dissout un composé de formule I dans une solution aqueuse d'un sel de formule II.

**17.** Procédé selon l'une des revendications 1 à 8, contenant du Propiconazol en tant que composé de formule I.

**Revendications pour l'Etat contractant suivant : ES**

**1.** Procédé de préparation d'un produit microbicide consistant en un mélange synergétique d'un composé de formule I

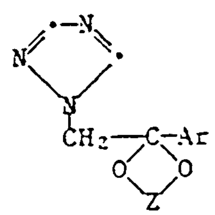

I

ou d'un sel de ce composé formé par addition avec un acide,
Z représentant $-CH_2CH_2-$, $-CH_2CH_2CH_2-$, $-CH(CH_3)-CH(CH_3)-$ou $-CH_2-CH$(alkyle en C 1-C 10)-, et
Ar un groupe thiophényle, halogénothiophényle, naphtalényle ou un groupe phényle non substitué ou portant éventuellement 1 à 3 substituants choisis parmi les halogènes, les groupes alkyle en C 1-C 4, alcoxy en C 1-C 4, cyano ou nitro,
et d'une quantité synergétique efficace d'un sel de formule II

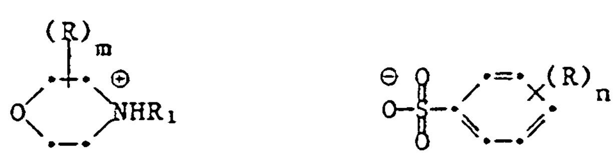

II,

dans laquelle
chacun des symboles R, indépendamment des autres, représente un groupe alkyle en C 1-C 4,
$R^1$ représente l'hydrogène ou un groupe alkyle en C 1-C 4, m est égal à 0, 1, 2, 3 ou 4, et
n est égal à 0, 1, 2, 3 ou 4,
caractérisé en ce que l'on mélange intimement les substances actives de formules I et II avec des produits auxiliaires, des véhicules et/ou des solvants, sous réserve que le solvant ne peut consister en

eau lorsque, dans la formule II, $R^1$ représente l'hydrogène et m est égal à 0.

**2.** Procédé selon la revendication 1, avec utilisation du
1-[2-(2,4-dichlorophényl)-1,3-dioxolanne-2-ylméthyl]-1H-1,2,4-triazole,
1-[2-(2,4-dichlorophényl)-4-méthyl-1,3-dioxolanne-2-ylméthyl]-1H-1,2,4-triazole,
1-[2-(2,4-dichlorophényl)-4-éthyl-1,3-dioxolanne-2-ylméthyl]-1H-1,2,4-triazole,
1-[2-(2,4-dichlorophényl)-4-propyl-1,3-dioxolanne-2-ylméthyl]-1H-1,2,4-triazole (Propiconazol), ou
1-[2-(2,4-dichlorophényl)-4-pentyl-1,3-dioxolanne-2-ylméthyl]-1H-1,2,4-triazole,
en tant que composé de formule I.

**3.** Procédé selon la revendication 1 ou 2, avec utilisation du
benzène-sulfonate de morpholine,
toluène-sulfonate de morpholine,
cumène-sulfonate de morpholine,
benzène-sulfonate de N-méthylmorpholine,
cumène-sulfonate de N-méthylmorpholine,
benzène-sulfonate de 2-méthylmorpholine,
toluène-sulfonate de 2-méthylmorpholine,
xylène-sulfonate de 2-méthylmorpholine,
benzène-sulfonate de 2,6-diméthylmorpholine,
xylène-sulfonate de 2,6-diméthylmorpholine,
cumène-sulfonate de 2,2,6-triméthylmorpholine, ou
cumène-sulfonate de 2,2,6,6-tétraméthylmorpholine,
en tant que composé de formule II.

**4.** Procédé selon l'une des revendications 1 à 3, caractérisé en ce que, outre les substances actives I et II, on mélange intimement et/ou on dissout une autre substance active agrochimique avec les produits auxiliaires, véhicules et/ou solvants.

**5.** Procédé selon la revendication 4, caractérisé en ce que l'on utilise en tant qu'autres substances agrochimiques
a) un engrais, des oligo-éléments ou d'autres produits agissant sur la croissance des végétaux,
b) un herbicide sélectif,
c) un fongicide,
d) un bactéricide,
e) un nématocide, ou
f) un mollusquicide.

**6.** Procédé selon la revendication 5, caractérisé en ce que l'on utilise en tant qu'autres fongicides le Tridemorph et/ou le Carbendazim.

**7.** Procédé selon l'une des revendications 1 à 6, caractérisé en ce que l'on dissout jusqu'à 75 % en poids/volume d'un composé de formule I dans une solution aqueuse d'un sel de formule II à une concentration allant jusqu'à 70 % en poids/volume.

**8.** Procédé pour le traitement des maladies des végétaux causées par des infections microbiennes, caractérisé en ce que l'on fait agir un produit obtenu par le procédé selon l'une des revendications 1 à 7 sur la plante, des parties de la plante, des organes de reproduction de la plante ou l'habitat de la plante.

**9.** Procédé selon la revendication 8, pour traiter des plantes cultivées.

**10.** Procédé selon la revendication 9, pour traiter des céréales.

**11.** Procédé selon l'une des revendications 8 à 10, caractérisé en ce que l'on applique le produit microbicide à une dose de 50 g à 5 kg de substance active par ha.

**12.** Procédé de préparation des sels de formule II

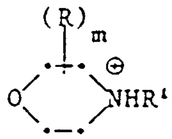 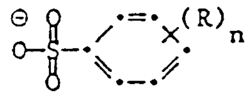 II,

dans laquelle
chacun des symboles R, indépendamment des autres, représente un groupe alkyle en C 1-C 4,
$R^1$ représente l'hydrogène ou un groupe alkyle en C 1-C 4,
m est égal à 0, 1, 2, 3 ou 4, et
n est égal à 0, 1, 2, 3 ou 4,
sous réserve que
le benzène-sulfonate de 2,6-diméthylmorpholine,
l'o-toluène-sulfonate de 2,6-diméthylmorpholine,
le m-toluène-sulfonate de 2,6-diméthylmorpholine,
le p-toluène-sulfonate de 2,6-diméthylmorpholine,
le 2,4,6-triméthylbenzène-sulfonate de 2,6-diméthylmorpholine,
le benzène-sulfonate de N-butylmorpholine,
l'o-toluène-sulfonate de N-butylmorpholine,
le m-toluène-sulfonate de N-butylmorpholine,
le p-toluène-sulfonate de N-butylmorpholine, et
le 2,4,6-triméthylbenzène-sulfonate de N-butylmorpholine sont exclus, caractérisé en ce que l'on fait réagir une morpholine de formule III

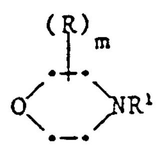 III,

dans laquelle les symboles $R^1$, A et m ont les significations indiquées ci-dessus,
avec un acide sulfonique de formule IV

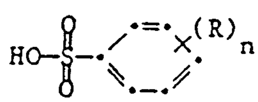 IV,

dans laquelle R et n ont les significations indiquées ci-dessus.

**13.** Procédé selon la revendication 12, pour la préparation du
cumène-sulfonate de morpholine,
benzène-sulfonate de N-méthylmorpholine,
cumène-sulfonate de N-méthylmorpholine,
benzène-sulfonate de 2-méthylmorpholine,
toluène-sulfonate de 2-méthylmorpholine,
cumène-sulfonate de 2-méthylmorpholine,
cumène-sulfonate de 2,6-diméthylmorpholine,
cumène-sulfonate de 2,2,6-triméthylmorpholine,
cumène-sulfonate de 2,2,6,6-tétraméthylmorpholine.